(19) Europäisches Patentamt
European Patent Office
Office européen des brevets

(11) Publication number: **0 062 019**
**A1**

(12) # EUROPEAN PATENT APPLICATION

(21) Application number: 82850057.9

(22) Date of filing: 22.03.82

(51) Int. Cl.³: C 07 D 223/04
A 61 K 31/55
//C07C101/28, C07C121/75

(30) Priority: 01.04.81 SE 8102077

(43) Date of publication of application:
06.10.82 Bulletin 82/40

(84) Designated Contracting States:
AT BE CH DE FR GB IT LI LU NL SE

(71) Applicant: Aktiebolaget Hässle
S-431 83 Mölndal(SE)

(72) Inventor: Arvidsson, Folke Lars-Erik
Seminariegränd 3
S-752 28 Uppsala(SE)

(72) Inventor: Carlsson, Per Arvid Emil
Torild Wulffsgatan 50
S-413 19 Göteborg(SE)

(72) Inventor: Engel, Jörgen Andrew B.
Askims stenlid 2
S-436 00 Askim(SE)

(72) Inventor: Hacksell, Uli Alf
Bandstolsvägen 4VI
S-752 48 Uppsala(SE)

(72) Inventor: Hjorth, John Stephan Mikael
Blävalsgatan 7A
S-414 75 Göteborg(SE)

(72) Inventor: Lindberg, Per Lennart
Knapehall 64
S-436 00 Askim(SE)

(72) Inventor: Mattsson, Annie Hillevi
Pilegärden 9C
S-436 00 Askim(SE)

(72) Inventor: Nilsson, John Lars Gunnar
Tullinge Strand 30B
S-146 00 Tullinge(SE)

(72) Inventor: Sanchez, Domingo
Snipäsvägen 24
S-440 03 Floda(SE)

(72) Inventor: Svensson, Nils Uno Eimer
Rallarvägen 6
S-740 20 Brunna(SE)

(72) Inventor: Wikström, Häkan Vilhelm
Gibsons väg 25
S-433 61 Partille(SE)

(74) Representative: Wurm, Bengt Runio et al,
Patent and Trade Mark Department Ab Astra
S-151 85 Södertälje(SE)

(54) ## New phenyl-perhydroazepines.

(57) Compounds of the formula

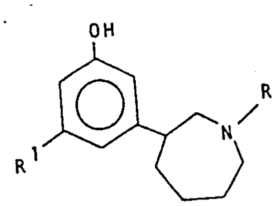

1

wherein R is an alkyl, hydroxyalkyl or alkenyl group and R¹ is hydrogen or a hydroxy group, processes for their preparation and pharmaceutical preparations and methods of treatment employing such compounds. The compounds are useful for therapeutic purposes, especially for treatment of cardiac diseases.

EP 0 062 019 A1

# New phenyl-perhydroazepines

## DESCRIPTION

### Technical field

The present invention is related to new substituted phenyl-piperidines, to processes for preparing such compounds as well as to pharmaceutical preparations thereof and methods of treatment employing such compounds.

An object of the invention is to provide compounds for therapeutic use, especially having a therapeutic activity as cardiac agents.

### Background Art

In Chemical Abstracts 69: 86776s (citing Julia, M. et al., Bull. Soc. Chim. Fr. 1968, (3), 1000-7) compounds under the general formula

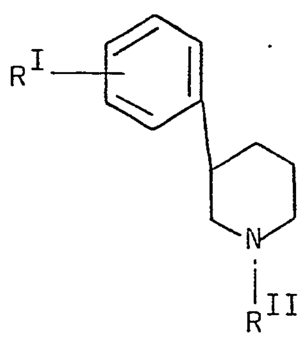

are described. Among the compounds mentioned are compounds wherein $R^I$ represents m-$OCH_3$ and $R^{II}$ represents H, $CH_3$, $C_2H_5$, $CH_2C_6H_5$, $CH_2CH_2C_6H_5$ or $CH_2CH_2C_6H_4NO_2$(p) and wherein $R^I$ represents m-OH and $R^{II}$ represents $CH_2CH_2C_6H_5$ or $CH_2CH_2C_6H_4NO_2$(p). Said compounds were prepared for investigation of pharmacological properties.

Swiss Patent 526,536 describes compounds under the formula

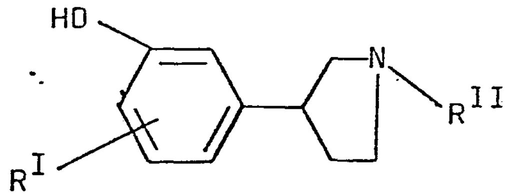

wherein $R^I$ represents H or OH and $R^{II}$ represents H. The compounds are claimed to have useful pharmacological properties especially as broncholytic agents.

DE-OLS 2 621 535 and 2 621 536 describe compounds of the formula

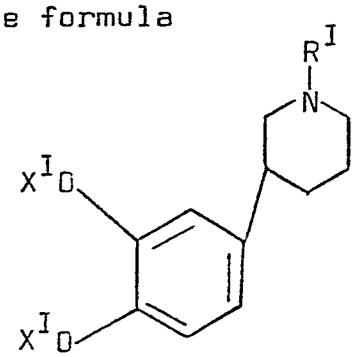

wherein $X^I$ is hydrogen or an acyl group and $R^I$ is H or an alkyl, alkenyl or phenylalkyl group. The compounds are claimed to have dopaminergic properties.

Disclosure of Invention

According to the present invention it has been found that novel compounds of the formula

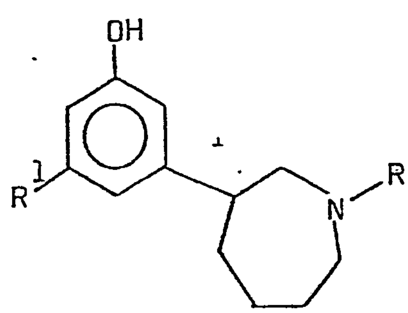

I

wherein R is an alkyl group having 1-5 carbon atoms, a hydroxyalkyl group having 2-6 carbon atoms in the alkyl part and having the hydroxy group bound in a position other than the 1 position, or an alkenyl group having 3-5 carbon atoms other than a 1-alkenyl group, and $R^1$ is hydrogen or a hydroxy group, as bases and pharmaceutically acceptable acid addition salts thereof, have useful pharmacological properties. Especially, the compounds are useful for treatment of cardiac diseases.

Thus, the compounds of formula I have an ability of increasing the contractility of the heart muscle of an animal. Thus, the compounds have a positive inotropic cardiac effect, rendering them useful for treatment of cardiac diseases in animals including man. Further, among the compounds of formula I certain compounds are expected to have a positive inotropic cardiac effect, substantially lacking positive chronotropic effect. The compounds of formula I are thus especially useful for treatment of cardiac insufficiency or cardiac failure and other conditions wherein an increase in heart contractility is desired.

In the compounds of the invention an alkyl group may be a straight alkyl group or a branched alkyl group having at least 3 carbon atoms. Symbols for numbers, atoms or groups referred to below have the broadest meaning previously assigned unless specified otherwise.

Both organic and inorganic acids can be employed to form non-toxic pharmaceutically acceptable acid addition salts of the compounds of this invention. Illustrative acids are sulfuric, nitric, phosphoric, hydrochloric, citric, acetic, lactic, tartaric, pamoic, ethanedisulfonic, sulfamic, succinic, cyclohexylsulfamic, fumaric,

maleic, and benzoic acid. These salts are readily prepared by methods known in the art.

Compounds of the invention to be specifically mentioned are 3-(3-hydroxyphenyl)N-n-propylperhydroazepine and 3-(3,5-dihydroxyphenyl)N-n-propylperhydroazepine, and their salts.

The compounds of the invention contain an asymmetric carbon atom in the heterocyclic ring moiety. The therapeutic properties of the compounds may to a greater or lesser degree be ascribed to either or both of the two enantiomers occurring. Thus, the pure enantiomers as well as mixtures thereof are within the scope of the invention.

The invention takes into consideration that compounds which structurally deviate from the formula I, after administration to a living organism may be transformed to a compound of the formula I and in this structural form exert their effects. This consideration is a further aspect of the invention.

Methods of Preparation

The compounds of the invention may be obtained by one of the following methods constituting a further aspect of the invention.

a) An ether or ester of the formula

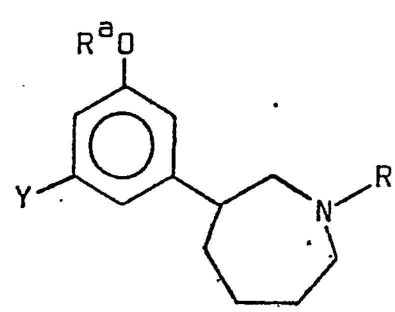

II

wherein $R^a$ represents a hydrocarbon or acyl residue, preferably an alkyl group having 1-5 carbon atoms, or an alkylcarbonyl group having 2-6 carbon atoms, Y represents hydrogen or the group $R^aO$ and R is as defined above, may be cleaved to form a compound of formula I.

When $R^a$ is a hydrocarbon residue the cleavage may be carried out by treating the compound of formula II with an acidic nucleophilic reagent such as aqueous HBr, or HI, $HBr/CH_3COOH$, $BBr_3$, $AlCl_3$, pyridine-HCl or $(CH_3)_3SiI$, or with a basic nucleophilic reagent such as $CH_3C_6H_4-S^{\ominus}$ or $C_2H_5-S^{\ominus}$.

When $R^a$ is an acyl residue the cleavage may be carried out by hydrolysis in an aqueous acid or base or by reduction, preferably by $LiAlH_4$.

The compound of formula II is obtainable by reacting a compound of formula IIA,

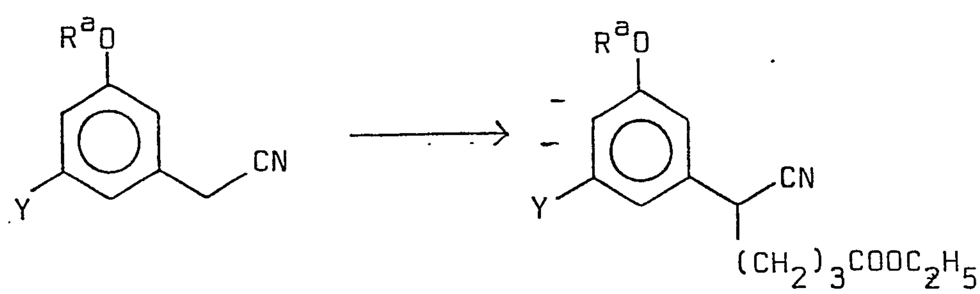

IIA IIB

wherein $R^a$ is an alkyl group, with $X^2(CH_2)_3COOC_2H_5$ ($X^2$=B,I) or $CH_2=CHCH_2-COOC_2H_5$, in the presence of a base, to the formation of a compound of formula IIB. The compound IIB is then converted into a compound of formula IID along the following route.

IIB 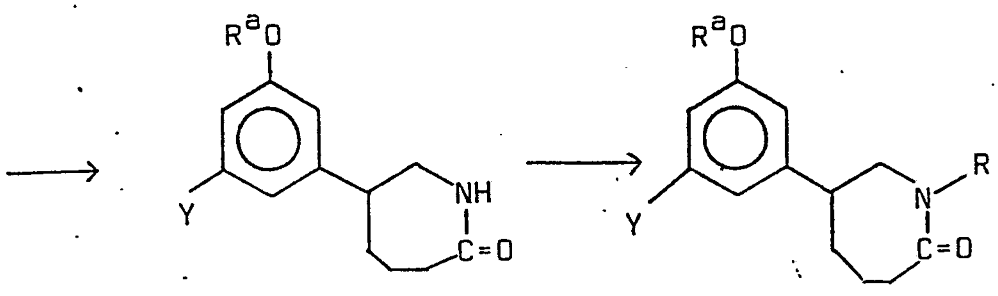

IIC IID

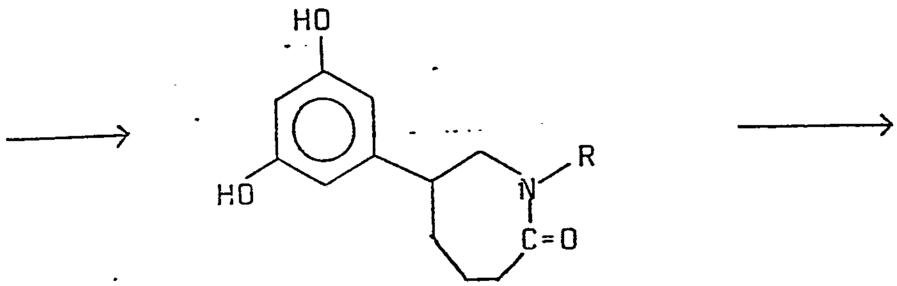 IID

Compound IIB is treated with hydrogen in the presence of a catalyst such as Raney nickel to the obtention of compound IIC, in which a substituent may be introduced at the nitrogen atom by means of a halide RX. If exchange of the group $R^a$ is desired the ether function is then cleaved with $BBr_3$ and the hydroxy group is acylated or realkylated in the presence of a base.

The amide compound of the formula IID is converted into a compound of the formula II by reduction of the amide function and, if required, introduction of a group R in the manner described under c) below. Thus the compound of formula IID may be treated with a reducing agent, preferably a hydride reducing agent such as $LiAlH_4$ or $BH_3$ in an etheral solvent or a metal reducing agent such as Na in an alcoholic solvent such as n-butanol. The ethers or esters according to formula II may further be obtained by several processes analogous to the processes for preparation of the mono- or di-hydroxyphenyl derivatives of formula I described below.

b) In a compound of the formula

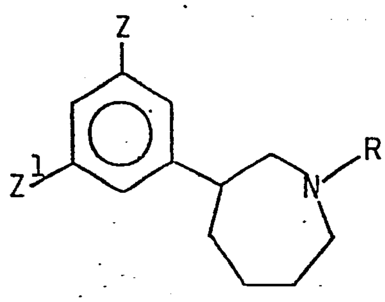

III

wherein Z represents $SO_3H$, Cl or $NH_2$, and $Z^1$ represents hydrogen or Z a hydroxy group may be substituted for each group Z to the formation of a compound of formula I. When Z is $SO_3H$ or Cl said reaction may be carried out by treatment with a strong alkali under heating, suitably with an alkali melt such as KOH when Z is $SO_3H$, and with a strong aqueous alkali such as NaOH or KOH when Z is Cl. When Z is $NH_2$ the reaction may be carried out by treatment with aqueous nitrous acid to the formation of an intermediate diazonium compound which is then subjected to hydrolysis in water.

c) A compound of the formula

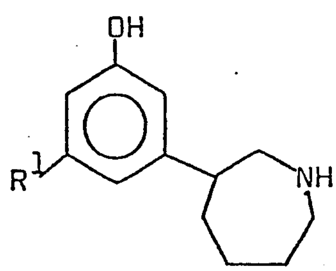

IV

may be converted into a compound of formula I by alkylation of the nitrogen atom with an appropriate alkylating agent. Thus, the starting compound may be treated with an

alkyl, hydroxyalkyl, or alkenyl halide or tosylate $RX^1$, wherein $X^1$ represents Cl, Br, I or

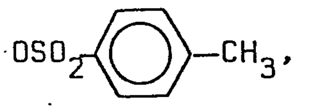, in an organic solvent such as acetonitrile or acetone and in the presence of a base such as $K_2CO_3$ or NaOH, or with an alcohol ROH in the presence of a catalyst, or the starting compound may be treated with a carboxylic acid $NaBH_4$ complex $R^bCOOH-NaBH_4$, wherein $R^b$ is defined by the relation $R^bCH_2-$ equals R. Further, the compound of formula IV may be reacted with an aldehyde of the formula $R^bCHO$, wherein $R^b$ is as just defined, and reduced to the formation of a compound of formula I. To the formation of a compound of formula I wherein R is $CH_3$, which is not obtainable by the last-mentioned reactions, the alkylation reaction may be carried out by treatment with a formaldehyde $-Na(CN)BH_3$ mixture. To the formation of a compound of formula I wherein R is hydroxylalkyl, the synthesis may also be carried out by alkylation with an appropriate dihaloalkane giving a monohaloalkyl derivative of I, followed by acid or alkaline hydrolysis. Especially, to the formation of a compound of formula I wherein R is 2-hydroxyalkyl, the alkylation may also be carried out by reaction with a 1,2-epoxyalkane.

d) A carbonyl compound of the formula

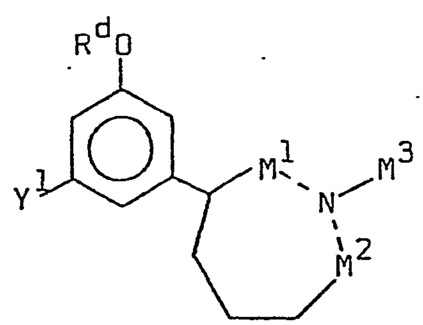

V

wherein $M^1$ and $M^2$ are the same or different and each

represents $-CH_2-$ or $>C=O$ and the dashed lines represent bonds, one of which, when adjacent to a group $>C=O$, may be open and replaced by hydrogen atoms, and $M^3$ is $-\underset{\underset{O}{\|}}{C}-R^c$ when $M^1$ and $M^2$ are both $-CH_2-$, and in other cases $M^3$ is R. $R^c$ is H, an alkyl or alkoxyl group containing 1-4 carbon atoms, a hydroxyalkyl group containing 1-5 carbon atoms, or an alkenyl group containing 2-4 carbon atoms, $R^d$ is H or $R^1CO$, wherein $R^1$ is an alkyl group having 1-5 carbon atoms and $Y^1$ is hydrogen or a group $R^dO$, may be converted into a compound of the formula I by reduction of the amide or imide function, and the ester function $R^1COO$ if present.

Thus the compound of formula V may be treated with a reducing agent, preferably a hydride reducing agent such as $LiAlH_4$ or $BH_3$ in an etheral solvent or a metal reducing agent such as Na in an alcoholic solvent such as n-butanol when ring closure is not required. When one of the dashed lines in formula V represents an open bond, the reduction comprises ring closure in a compound of the formula

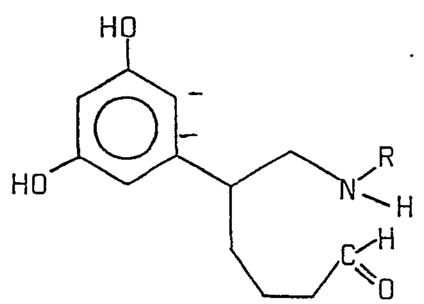

or

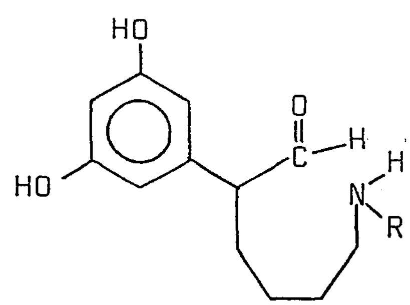

and may be done by catalytic hydrogenation.

e) A compound of the formula

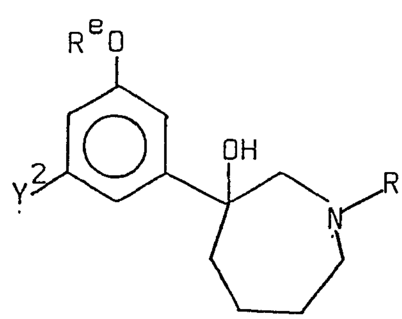

VI

wherein $R^e$ is H or benzyl and $Y^2$ is H or $R^eO$ and wherein R is an alkyl or hydroxyalkyl group as further defined above, may be converted either by direct reduction or by first elimination of the tertiary alcohol to an intermediary 1-cycloalkenyl compound and then reduction into a compound of formula I, wherein R is as just defined. The reduction may preferably be carried out by catalytic hydrogenation with a catalyst such as Pd or $PtO_2$, and the elimination reaction by heating in the presence of an acid.

f) An enamine of the formula

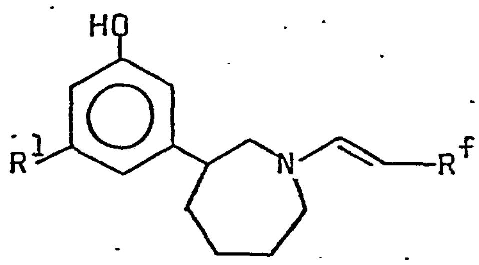

VII

wherein $R^f$ is defined by the relation $R^fCH_2CH_2$- equals R, may be converted by reduction into a compound of formula I wherein R is an alkyl group with 2-5 carbon atoms. The reduction may preferably be carried out by catalytic hydrogenation using a catalyst such as Pd or $PtO_2$.

g) A compound according to the formula

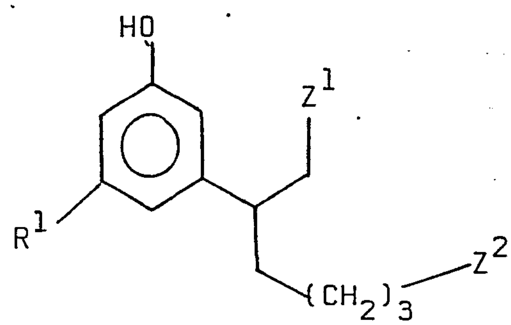

VIII

wherein one of the groups $Z^1$ and $Z^2$ is a leaving group X and the other is NHR, or $Z^1$ and $Z^2$ are both leaving groups X, and X is a leaving group such as Cl, Br, I or $-OSO_2C_6H_4CH_3$, may be converted to a compound of formula I by treating the compound of formula VIII, or when one of $Z^1$ and $Z^2$ is NHR an acid addition salt thereof, with a base such as $(C_2H_5)_3N$ or $K_2CO_3$, whereby the compound of formula VIII is treated together with an equivalent amount of an amine $R-NH_2$ or an acid addition salt thereof when $Z^1$ and $Z^2$ are both X. The conversion is carried out in a solvent such as tetrahydrofuran, dioxan or acetonitrile, if necessary, with simultaneous or subsequent heating of the mixture.

Free bases formed may subsequently be converted into their acid addition salts, and acid addition salts formed may subsequently be converted into the corresponding bases or other acid addition salts.

The new compounds may, depending on the choice of starting materials and process, be obtained as enantiomers or mixtures thereof. The enantiomeric mixtures or racemates obtained can be separated according to known methods, e.g. by means of recrystallization from an optically active solvent, by means of microorganisms, or by a reaction with optically active acids forming salts of the compound and separating the salts thus obtained, e.g. by means of their different solubility in the diastereomers, from which the antipodes by the influence of a suitable agent may be set free. Suitably useable optically active acids are e.g. the L- and D-forms of tartaric acid, di-o-tolyltartaric acid, malic acid, mandelic acid, camphersulfonic acid or quinic acid. Preferably the more active part of the two antipodes is isolated.

Starting materials for the methods of preparation described above may be obtained by several methods known in the art.

Pharmaceutical preparations

Pharmaceutical preparations of the compounds of the invention constitute a further aspect of the invention.

In clinical practice the compounds of the present invention will normally be administered orally, rectally, or by injection, in the form of pharmaceutical preparations comprising the active ingredient either as a free base or as a pharmaceutically acceptable non-toxic, acid addition

salt, e.g. the hydrochloride, lactate, acetate, sulfamate, and the like, in association with a pharmaceutically acceptable carrier.

Accordingly, terms relating to the novel compounds of this invention, whether generically or specifically, are intended to include both the free amine base and the acid addition salts of the free base, unless the context in which such terms are used, e.g. in the specific examples, would be inconsistent with the broad concept. The carrier may be a solid, semisolid or liquid diluent or capsule. These pharmaceutical preparations constitute a further aspect of this invention. Usually the active substance will constitute between 0.1 and 99% by weight of the preparation, more specifically between 0.5 and 20% by weight for preparation intended for injection and between 0.2 and 50% by weight for preparations suitable for oral administration.

Pharmaceutical preparations containing a compound of the invention in a solid form of dosage units for oral application may preferably contain between 2 and 50% by weight of the active substance, in such preparations the selected compound may be mixed with a solid fine grain carrier, e.g. lactose, saccharose, sorbitol, mannitol, starches such as potato starch, corn starch or amylopectin, cellulose derivatives, or gelatin and a lubricant such as magnesium stearate, calcium stearate, polyethylene glycol waxes, and the like, and then compressed to form tablets. If coated tablets are required, the cores, prepared as described above, may be coated with a concentrated sugar solution which may contain, e.g. gum arabic, gelatin, talcum, titanium dioxide, and the like. Alternatively the tablet can be coated with a lacquer dissolved in a readily volatile organic solvent or mixture of organic solvents. Dyestuffs may be added to these coatings in order to readily distinguish between tablets containing

different active substances or different amounts of the active compound.

For the preparation of soft gelatin capsules (pearl-shaped closed capsules) consisting of gelatin and, for example, glycerol, or similar closed capsules, the active substance may be admixed with a vegetable oil. Hard gelatin capsules may contain granulates of the active substance in combination with solid, fine grain carriers such as lactose, saccharose, sorbitol, mannitol, starches (e.g. potato starch, corn starch or amylopectin), cellulose derivatives or gelatin.

Liquid preparations for oral application may be in the form of syrups or suspensions, for example, solutions containing from about 0.2% to about 20% by weight of the active substance herein described, the balance being sugar and a mixture of ethanol, water, glycerol and propyleneglycol. Optionally such liquid preparations may contain colouring agents, flavouring agents, saccharine and carboxymethylcellulose as a thickening agent.

Solutions for parenteral applications by injection can be prepared in an aqueous solution of a water-soluble pharmaceutically acceptable salt of the active substance preferably in a concentration of from about 0.5% to about 10% by weight. Thsee solutions may also contain stabilizing agents and/or buffering agents and may conveniently be provided in various dosage unit ampoules.

In therapeutical treatment the suitable daily doses of the compounds of the invention are 200-10000 mg for oral application, preferentially 1000-6000 mg, and 1-1000 mg for parenteral application, preferentially 50-500 mg.

Working examples

Example 1

3-(3-Hydroxyphenyl)N-n-propylperhydroazepine

3-(3-Methoxyphenyl)N-n-propylperhydroazepine · HCl (0.7 g) was dissolved in 48 % HBr (20 ml) and heated at 120°C for 2 hours under $N_2$. Evaporation of the HBr *in vacuo* and purification of the aminophenol by extractions followed by precipitation of the compound as the hydrochloride, afforded 3-(3-hydroxyphenyl)-N-n-propylperhydroazepine 0.43 g (62 %),mp. 148-50°C. Anal. ($C_{15}H_{29}ClNO$), C, H, N.

The starting material was prepared by the following procedure.

Ethyl-5-cyano-5(3-methoxyphenyl)pentanoate

3-Methoxyphenylacetonitrile (35 g, 238 mmol) in THF (50 ml) was added to a freshly prepared solution of lithium di-*iso*-propylamide (238 mmol) in THF (100 ml) under argon. The mixture was kept at -70°C for 1.5 h, whereafter a solution of ethyl-1-iodobutyrate (58 g, 238 mmol) in THF (50 ml) was added. After another hour at -70°C the mixture was allowed to reach room temperature. Addition of 10 % HCl (100 ml) followed by separation and evaporation of the organic layer gave an oily residue which was partitioned between $H_2O$ and $CH_2Cl_2$. The organic layer was separated dried ($Na_2SO_4$) and evaporated, affording an oil which was distilled *in vacuo* yielding 39 g (63 %) of ethyl-5-cyano--5-(3-methoxyphenyl)pentanoate, b.p. 175-185°C (1 mmHg).

Ethyl-6-Amino-5-(3-methoxyphenyl)hexanoate

A solution of ethyl-5-cyano-5(3-methoxyphenyl)pentanoate (10 g, 38.3 mmol) in EtOH (300 ml) was hydrogenated at 50 psi over Raney Ni (15 g) in a Parr apparatus. Removal of the catalyst by filtration followed by evaporation of the EtOH gave an oil which was dissolved in ether and treated with etheral HCl yielding 7.7 g (67 %) of ethyl-6-amino-5-(3-methoxyphenyl)hexanoate as the hydrochloride, mp. 90-92$^{o}$C.

3-(3-Methoxyphenyl)perhydroazepine

A mixture of ethyl-6-amino-5-(3-methoxyphenyl)hexanoate · HCl (7.0 g, 23.2 mmol) and 20 % NaOH (150 ml) was refluxed for 1 hour. The reaction mixture was acidified with conc. HCl and evaporated. The oily residue was taken up in EtOH, the NaCl filtered off and the EtOH was evaporated. The amino acid hydrochloride thus obtained was dissolved in $CH_2Cl_2$ (75 ml), thionyl chloride (10 ml) was added and the resulting solution was stirred at ambient temperature for 1 hour. Evaporation of the volatiles afforded an oily residue which was dissolved in toluene (30 ml) and triethylamine (10 ml). The resulting solution was refluxed for 48 hours, the volatiles were evaporated and the residue was partitioned between EtOH and $H_2O$. The organic layer was dried ($Na_2SO_4$) and evaporated, affording 4 g of the crude lactam. This was dissolved in THF (40 ml) and added to a suspension of $LiAlH_4$ (1.0 g, 26 mmol) in THF (50 ml) under $N_2$. After reflux for 1 hour the reaction mixture was hydrolyzed, the precipitate was filtered off and the THF was evaporated. The resulting oil (2.8 g) was chromatographed on a silica column eluated with MeOH yielding 0.8 g (20 %) of 3-(3-methoxyphenyl)perhydroazepine as an oil.

3-(3-Methoxyphenyl)N-n-propylperhydroazepine

Sodium borohydride (0.76 g) was added to a solution of propionic acid (4.62 g, 63 mmol) in benzene (30 ml) and the mixture was kept at ambient temperature for 12 hours. 3-(3-Methoxyphenyl)perhydroazepine (0.8 g, 4.5 mmol) in benzene (20 ml) was added and the solution refluxed for 3 hours. 2.5N NaOH (30 ml) was added and the phases were separated, the organic layer dried ($Na_2SO_4$) and evaporated. The crude tertiary amine was chromatographed on a silica column with MeOH as eluant. The oily residue was dissolved in ether and treated with etheral HCl affording 0.7 g (62 %) of 3-(3-methoxyphenyl)N-n-propylperhydroazepine as an oil.

Example 2

3-(3,5-Dihydroxyphenyl)N-n-propylperhydroazepine hydrobromide

3-(3,5-Dimethoxyphenyl)N-n-propylperhydroazepine hydroiodide (5,9 g, 14,5 mmol) was demethylated in hydrobromic acid (65 ml, constant boiling) giving 1.9 g white crystals after purification from a mixture of methanol and acetonitrile. Mp. 185°C, NMK ($^1$H and $^{13}$C) according to structure.

The starting material was prepared by the following procedure.

Ethyl-6-amino-5-(3,5-dimethoxyphenyl)hexanoate

Prepared according to Example 1 from ethyl-5-cyano-5(3,5-dimethoxyphenyl)pentanoate yield 77%.

3-(3,5-Dimethoxyphenyl)perhydroazepine hydrochloride

Prepared according to Example 1 from ethyl-6-amino-5-(3,5-dimethoxyphenyl)hexanoate.

3-(3,5-Dimethoxyphenyl)N-n-propylperhydroazepine hydroiodide

n-Propyliodide (3,4 g, 20 mmol) was added to a reflexing solution of 3-(3,5-dimethoxyphenyl)perhydroazepine (4,3 g, 18 mmol) in acetonitrile (45 ml). After reflux for 2 hours, the solution was evaporated and the residue treated with ethylacetate and filtreted, giving 6,0 g (82%) white crystals. Mp. 165°C, NMK ($^1$H) according to structure.

The following examples illustrate how the compounds of the present invention may be included into pharmaceutical preparations.

Example 3. Preparation of soft gelatine capsules

500 g of active substance are mixed with 500 g of corn oil, whereupon the mixture is filled in soft gelatine capsules, each capsule containing 100 mg of the mixture (i.e. 50 mg of active substance).

Example 4. Preparation of tablets

0.5 kg of active substance are mixed with 0.2 kg of silicic acid of the trade mark Aerosil. 0.45 kg of potato starch and 0.5 kg of lactose are mixed therewith and the mixture is moistened with a starch paste prepared from 50 g of potato starch and distilled water, whereupon the mixture is granulated through a sieve. The granulate is dried and sieved, whereupon 20 g of magnesium stearate are mixed into it. Finally the mixture is pressed into tablets each weighing 172 mg.

Example 5. Preparation of a syrup

100 g of active substance are dissolved in 300 g of 95 % ethanol, whereupon 300 g of glycerol, aroma and colouring agents (q.s.) and 1000 ml of water are mixed therein. A syrup is obtained.

Example 6. Preparation of an injection solution

Active substance (hydrobromide), (1 g), sodiumchloride (0.8 g) and ascorbic acid (0.1 g) are dissolved in sufficient amount of distilled water to give 100 ml of solution. This solution, which contains 10 mg of active substance per ml, is used in filling ampoules, which are sterilized by heating at 120°C for 20 minutes.

Pharmacological studies on cardiac effects on heart frequency and contractility in isolated right atria and papillary muscle from rabbit.

Rabbits with an average weight of 2.5 kg, pretreated with reserpine (5 mg/kg i.v.) about 16 hours before the experiment were used. The rabbits were killed by a blow and the hearts were rapidly removed. The right atria and a papillary muscle from the right ventricle were isolated and set up to contract isometrically in an organ bath. The bath contained 50 ml Krebs-buffer (pH 7.4) and the temperature was kept at 32°C.

The contractions were measured by means of a force displacement transducer (Grass Model FT03) coupled to a Grass Model 7 Polygraph with a preampliphier (Grass Model 7PIF).

The spontaneously beating right atria was stretched to a resting tension high enough to measure the contractions and to trigger a tachograph for registration of the frequency. The papillary muscle was stretched to the length at which maximal tension was developed and was driven at a frequency of 1HZ by a current 20 per cent above threshold and with a duration of 5 msec.

After 60 min. equilibration, dose response curves to cumulative concentrations of isoprenaline were constructed for chronotropic effect, i.e. increase in frequency (atria) and positive inotropic effect, i.e. increase in contractility (papillary muscle). After wash-out similar curves were constructed for the compounds employed according to the invention. The positive inotropic agent dopamine was included for comparison.

In order to establish the maximal chronotropic and inotropic responses of the preparations, isoprenaline was added immediately after the maximal concentrations of the compounds. The maximal effects of the compounds compared with the maximal effect of isoprenaline are expressed as intrinsic activity (ISA).

Results

As can be seen from Table I all the compounds tested produced an increase in contractility, i.e. displayed a positive inotropic effect in the isolated rabbit right papillary muscle.

The positive inotropic effect of the tested compound of the invention is (when the ISA-values are compared) somewhat lower than that obtained with dopamine. However, the $EC_{50}$ for contractility for the compound of Example 1 was only about one third of the $EC_{50}$ for dopamine.

TABLE I

The effect of certain compounds on rabbit right atria (heart rate, HR) and rabbit right ventricle papillary muscle (contractility, $T_{max}$)

| Compound | Salt/Base | HR $EC_{50}$ µM | ISA % | $T_{max}$ $EC_{50}$ µM | ISA % | n |
|---|---|---|---|---|---|---|
| Isoprenaline | bitartrate | 0.003 | 100 | 0.01 | 100 | |
| Dopamine | HCl | 38 | 97 | 36 | 77 | 2 |
| Example 1 | HBr | 0.35 | 31 | 11 | 37 | 4 |
| " 2[1)] | | - | 0 | 2 | 31 | 2 |

1) not pretreated with reserpine

Conclusion

The tested compounds according to the invention displayed strong positive inotropic properties in the isolated rabbit papillary muscle. Thus the compounds according to the invention are expected to have therapeutic potentials for cardiovascular diseases where a positive inotropic effect is desirable.

Best mode of carrying out the invention

The method of the invention employing 3-(3,5-dihydroxyphenyl)-N-n-propylperhydroazepine and its salts represents the best mode of carrying out the invention known to the inventors at present. Other N-substituted 3-(3,5-dihydroxyphenyl)perhydroazepine compounds of the invention may also be very useful.

CLAIMS

1. A compound characterized by the formula

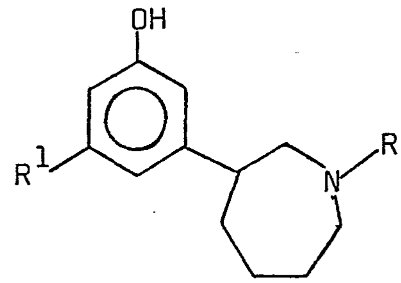

I

wherein R is an alkyl group having 1-5 carbon atoms, a hydroxyalkyl group having 2-6 carbon atoms in the alkyl part and having the hydroxy group bound in a position other than the 1 position, an alkenyl group having 3-5 carbon atoms other than a 1-alkenyl group, and $R^1$ is hydrogen or a hydroxy group, as the base or a pharmaceutically acceptable acid addition salt thereof.

2. A compound according to claim 1 characterized in that R is an alkyl group having 3-5 carbon atoms.

3. The compound 3-(3-hydroxyphenyl)N-n-propylperhydroazepine as the base or a pharmaceutically acceptable acid addition salt thereof.

4. The compound 3-(3,5-dihydroxyphenyl)-N-n-propylperhydroazepine as the base or a pharmaceutically acceptable acid addition salt thereof.

5. A process for preparation of a compound of the formula

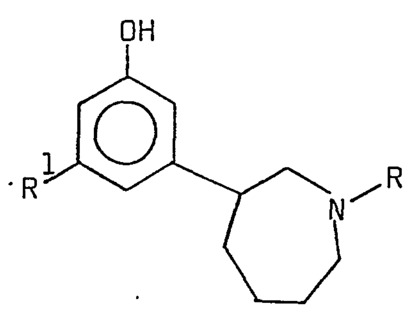

I

wherein R is an alkyl group having 1-5 carbon atoms, a hydroxyalkyl having 2-6 carbon atoms in the alkyl part and having the hydroxy group bound in a position other than the 1 position, an alkenyl group having 3-5 carbon atoms other than a 1-alkenyl group, and $R^1$ is hydrogen or a hydroxy group, as the base or pharmaceutically acceptable acid addition salt thereof, characterized in that

a) an ether or ester of the formula

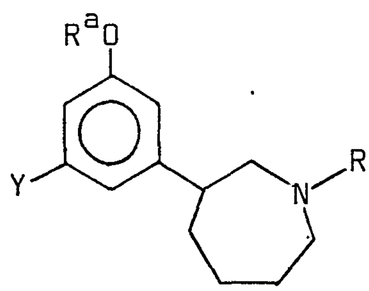

II

wherein $R^a$ represents a hydrocarbon or acyl residue, Y represents hydrogen or a group $R^aO$, and R is as defined above, is cleaved to form a compound of formula I or

b) in a compound of the formula

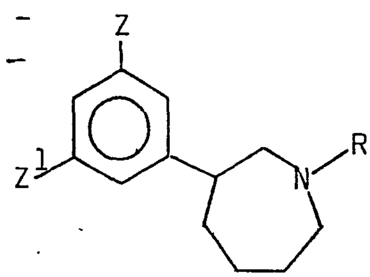

III

wherein Z represents $SO_3H$, Cl or $NH_2$, $Z^1$ represents hydrogen or a group Z, and R is as defined above, hydroxy groups are substituted for each group Z to the formation of a compound of formula I, or

c) a compound of the formula

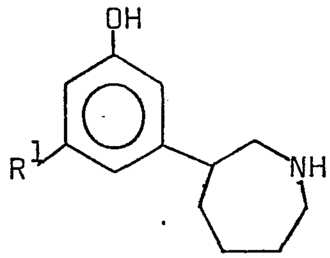

IV

is converted into a compound of formula I by alkylation of the nitrogen atom with an appropriate alkylating agent, or

d) a carbonyl compound of the formula

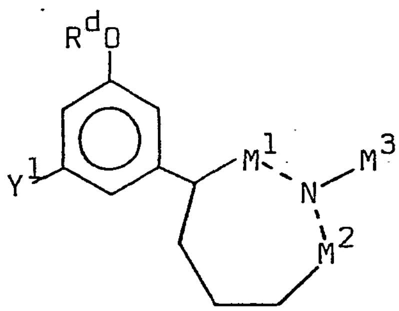

V

wherein $M^1$ and $M^2$ are the same or different and each represents $-CH_2-$ or $>C=O$ and the dashed lines represent bonds, one of which, when adjacent to a group $>C=O$, may be open and replaced by hydrogen atoms, and $M^3$ is $-\underset{\underset{O}{\|}}{C}-R^c$ when $M^1$ and $M^2$ are both $-CH_2-$, and in other cases $M^3$ is R, $R^c$ is H, an alkyl or alkoxyl group containing 1-4 carbon atoms, a hydroxyalkyl group containing 1-5 carbon atoms, or an alkenyl group containing 2-4 carbon atoms, $R^d$ is H, $R^1CO$, wherein $R^1$ is an alkyl group having 1-5 carbon atoms, and $Y^1$ is hydrogen or a group $R^dO$, is converted into a compound of the formula I by reduction, or

e) a compound of the formula

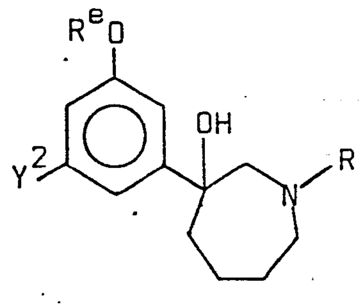

VI

wherein $R^e$ is H or benzyl and $Y^2$ is H or a group $R^eO$, and wherein R is an alkyl or hydroxyalkyl group as further defined above, is converted either by direct reduction or by first elimination of the tertiary alcohol to an intermediary 1-cycloalkenyl compound and then reduction into a compound of formula I, or

f) an enamine of the formula

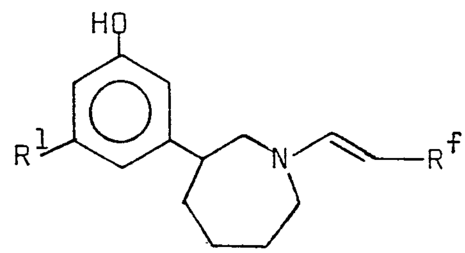

VII

wherein $R^f$ is defined by the relation $R^fCH_2CH_2$- equals R, is converted by reduction into a compound of formula I wherein R is an alkyl group with 2-5 carbon atoms, or

g) a compound according to the formula

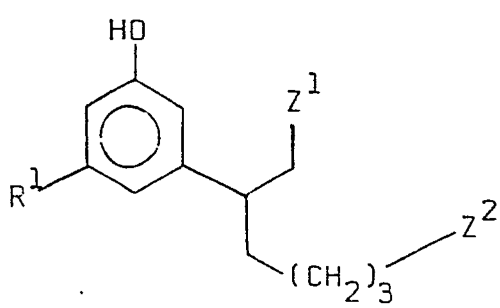

IX

wherein one of the groups $Z^1$ and $Z^2$ is a leaving group

and the other is NHR, or $Z^1$ and $Z^2$ are both leaving groups and R is as defined above, is converted to a compound of formula I by treating the compound of formula IX, or when one of $Z^1$ and $Z^2$ is NHR an acid addition salt thereof, with a base, whereby the compound of formula IX is treated together with an equivalent amount of an amine $R-NH_2$ or an acid addition salt thereof when $Z^1$ and $Z^2$ are both leaving groups;

whereupon optionally a base obtained is converted to a pharmaceutically acceptable acid addition salt or a salt obtained is converted to the base or to a different, pharmaceutically acceptable acid addition salt, and optionally an isomeric mixture obtained is separated to a pure isomer.

6. A process according to claim 4, characterized in that R is an alkyl group having 3-5 carbon atoms.

7. A process according to claim 5 characterized in preparing one of the compounds of claims 3 and 4.

8. A pharmaceutical preparation comprising as an active ingredient a compound according to one or more of claims 1 to 4, in conjunction with a pharmaceutically acceptable carrier.

0062019

European Patent Office

# EUROPEAN SEARCH REPORT

Application number

EP 82 85 0057

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | US-A-2 740 778 (J. DIAMOND) * Whole document * --- | 1,8 | C 07 D 223/04 A 61 K 31/55 // C 07 C 101/28 C 07 C 121/75 |
| A | GB-A-1 285 025 (J. WYETH) * Whole document * --- | 1,5,8 | |
| A | GB-A-1 319 785 (J. WYETH) * Whole document * --- | 1,8 | |
| D,A | DE-A-2 621 536 (ROUSSEL UCLAF) * Whole document * --- | 1,2,8 | |
| D,A | BULLETIN DE LA SOCIETE CHIMIQUE DE FRANCE, no. 3, 1968, published in Paris, FR. M. JULIA et al.: "No 156. Recherches sur les aryl-3 piperidines. II. Methoxy phenyl (o-, m-, p-), naphtyl et tetralyl-3 piperidines N-substituees" * Page 1001, table I; pages 1005-1007, experimental part * --- | 1,2,5,8 | TECHNICAL FIELDS SEARCHED (Int. Cl. 3) C 07 D 223/00 A 61 K 31/00 |

./.

The present search report has been drawn up for all claims

| Place of search | Date of completion of the search | Examiner |
|---|---|---|
| THE HAGUE | 28-06-1982 | NUYTS A.M.K.A. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03.82

0062019

Application number

European Patent Office

# EUROPEAN SEARCH REPORT

EP 82 85 0057



| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. 3) |
| P,X | JOURNAL OF MEDICINAL CHEMISTRY, vol. 24, December 1981, pages 1475-1482, Washington, USA U. HACKSELL et al.: "3-Phenylpiperidines. Central dopamine-autoreceptor stimulating activity" * Page 1478, table I, formula D; page 1479, compound 58; page 1482, left-hand column * ----- | 1-4,5, 8 | |
| | | | TECHNICAL FIELDS SEARCHED (Int. Cl. 3) |

The present search report has been drawn up for all claims

| Place of search | Date of completion of the search | Examiner |
|---|---|---|
| THE HAGUE | 28-06-1982 | NUYTS A.M.K.A. |

CATEGORY OF CITED DOCUMENTS

X particularly relevant if taken alone
Y particularly relevant if combined with another document of the same category
A technological background
O non-written disclosure
P intermediate document
T theory or principle underlying the invention
E earlier patent document, but published on, or after the filing date
D document cited in the application
L document cited for other reasons
& member of the same patent family, corresponding document

EPO Form 1503 03 82